(19) Europäisches Patentamt
European Patent Office
Office européen des brevets

(11) EP 0 408 973 B1

(12) EUROPEAN PATENT SPECIFICATION

(45) Date of publication and mention of the grant of the patent:
**23.10.1996 Bulletin 1996/43**

(51) Int Cl.[6]: **C07K 7/06**, A61K 38/08

(21) Application number: **90112643.3**

(22) Date of filing: **03.07.1990**

(54) **Antiherpes pentapeptides**

Antiherpes-Pentapeptide

Pentapeptides ayant une activité antiherpès

(84) Designated Contracting States:
**AT BE CH DE DK ES FR GB GR IT LI LU NL SE**

(30) Priority: **07.07.1989 CA 605092**

(43) Date of publication of application:
**23.01.1991 Bulletin 1991/04**

(73) Proprietor: **BIO-MEGA/BOEHRINGER INGELHEIM RESEARCH INC.**
**Laval Québec H7S 2G5 (CA)**

(72) Inventors:
- **Adams, Julian**
  **Ridgefield, Connecticut 06877 (US)**
- **Beaulieu, Pierre Louis**
  **Montréal, Québec, H2J 2Z9 (CA)**
- **Lavallée, Pierre**
  **Rosemére, Québec, J7A 4B2 (CA)**
- **Plante, Raymond**
  **Laval, Québec, H7L 4W8 (CA)**
- **Rakhit, Sumanas**
  **Dollard des Ormeaux, Québec, H9A 2A5 (CA)**

(74) Representative: **Laudien, Dieter, Dr. et al**
**Boehringer Ingelheim International GmbH**
**ZA Patente**
**Postfach 200**
**55216 Ingelheim am Rhein (DE)**

(56) References cited:
**EP-A- 0 246 630** **EP-A- 0 292 255**

- **THE JOURNAL OF BIOLOGICAL CHEMISTRY, vol. 262, no.26, pages 12413-12416.**
- **J. MED. CHEM., vol. 33, February 1990, pages 723-730.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 408 973 B1

## Description

### Field of the Invention

This invention relates to peptide derivatives having antiviral properties and to means for using the derivatives to treat viral infections. More specifically, the invention relates to peptide derivatives (hereinafter called "peptides") exhibiting activity against herpes viruses, to pharmaceutical compositions comprising the peptides, and to a method of using the peptides to treat herpes infections.

### Background of the Invention

The family of herpes viruses is responsible for a wide range of infections that afflict humans and many important domestic animals. The diseases caused by these viruses range from bothersome cold sores to highly destructive infections of the central nervous system (encephalitis). The more common members of this family include herpes simplex virus (types 1 and 2) responsible for cold sores and genital lesions; varicella zoster virus which causes chicken pox and shingles; and Epstein-Barr virus which causes infectious mononucleosis. Although some significant advances have been made in the last decade in antiviral therapy, the need for effective, safe therapeutic agents for treating herpes viral infections continues to exist. For a recent review of current therapeutic agents in this area, see M.C. Nahata, "Antiviral Drugs: Pharmacokinetics, Adverse Effects and Therapeutic Use", J. Pharm. Technol., 3, 100 (1987).

The present application discloses a group of peptide derivatives having activity against herpes viruses. The relatively selective action of these peptides against herpes viruses, combined with a wide margin of safety, renders the peptides as desirable agents for combating herpes infections.

The association of peptides with anti-herpes activity is uncommon. Instances of reports of such an association include B.M. Dutia et al., Nature, 321, 439 (1986), E.A. Cohen et al., Nature, 321, 441 (1986), J.H. Subak-Sharpe et al., UK patent application 2185024, published July 8, 1987, E.A. Cohen et al., European patent application 246630, published November 25, 1997, R. Freidinger et al., European patent application 292255, published November 23, 1988, and R. Freidinger et al., U.S. patent 4,814,432 issued March 21, 1989 Gaudreau P. et al., J. Biol. Chem, 262 (26), 12413-12416 (1987) disclosed peptides having antiviral activity against herpes viruses. These peptides were derived from the C-terminal of the H2 unit of the Herpes simplex virus ribonucleotide reductase (RR) and function by inhibition of this enzyme. Also disclosed was the discovery that acetylation of RR analogues led to increased inhibitor activity.

The subject peptides of the previous reports can be distinguished from the peptides of the present application by characteristic structural and biological differences.

### Summary of Invention:

The peptides of this invention are represented by formula 1

$$XNR^{1}\text{-}CH(R^{2})\text{-}C(O)\text{-}NH\text{-}CH(R^{3})\text{-}C(O)\text{-}NR^{4}\text{-}CH(CH_{2}Y)\text{-}CO\text{-}NH\text{-}$$
$$CH[CR^{5}(R^{6})\text{-}COOH]\text{-}C(W)\text{-}NH\text{-}CH\text{-}(R^{7})\text{-}Z \qquad 1$$

wherein X is (1-10C)alkanoyl, (1-10C)alkoxycarbonyl, benzoyl, benzoyl monosubstituted or disubstituted with a substituent selected from halo, hydroxy, lower alkyl, lower alkoxy, phenyl, 2-carboxyphenyl or benzyl, 2,2-diphenylacetyl, phenyl (2-10C)alkanoyl or phenyl(2-10C)alkonyl monosubstituted or disubstituted on the aromatic portion thereof with a substituent selected from halo, hydroxy, lower alkyl, lower alkoxy or phenyl;

$R^1$ is lower alkyl or phenyl(lower)alkyl;
$R^2$ is $CH(CH_3)_2$ or $CH(OH)CH_3$;
$R^3$ is $CH(CH_3)C_2H_5$;
$R^4$, $R^5$ and $R^6$ each is hydrogen;
$R^7$ is $CH_2CH(CH_3)_2$;
W is oxo or thioxo;
Y is carboxy, carbamyl or 5-1H-tetrazolyl; and
Z is carboxy or 5-1H-tetrazolyl; or a therapeutically acceptable salt thereof.

A preferred group of the peptides of this invention is represented in formula 1 wherein X and $R^1$ are as defined hereinabove, and $R^2$ is $CH(CH_3)_2$; $R^3$ is $CH(CH_3)C_2H_5$; $R^4$, $R^5$ and $R^6$ each is hydrogen, $R^7$ is $CH_2CH(CH_3)_2$; W is oxo; Y is carbamyl or 5-1H-tetrazolyl; and Z is carboxy or 5-1H-tetrazolyl; or a therapeutically acceptable salt thereof.

Included within the scope of this invention is a pharmaceutical composition comprising an anti-herpes virally effective amount of a peptide of formula 1, or a therapeutically acceptable salt thereof, and a pharmaceutically or veterinarily acceptable carrier.

Also included within the scope of this invention is a cosmetic composition comprising a peptide of formula 1, or a therapeutically acceptable salt thereof, and a physiologically acceptable carrier suitable for topical application.

An important aspect of the invention involves a method of treating herpes viral infections in a mammal by administering to the mammal an anti-herpes virally effective amount of the peptide of formula 1, of a therapeutically acceptable salt thereof.

Another important aspect involves a method of inhibiting the replication of herpes virus by contacting the virus with a herpes viral ribonucleotide reductase inhibiting amount of the peptide of formula 1, or a therapeutically acceptable salt thereof.

Processes for preparing the peptides of formula 1 are described hereinafter.

Details of the Invention

GENERAL

Alternatively, formula 1 can be illustrated as:

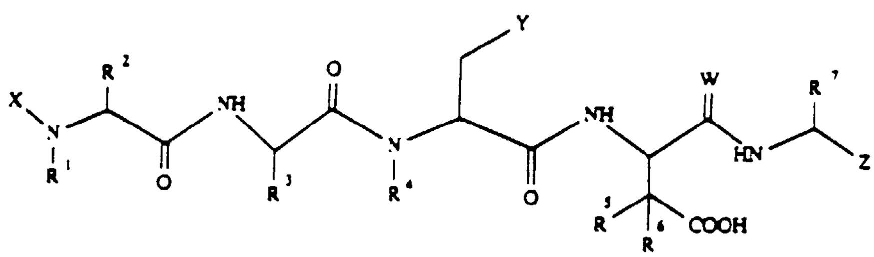

The term 'residue' with reference to an amino acid or amino acid derivative means a radical derived from the corresponding α-amino acid by eliminating the hydroxyl of the carboxy group and one hydrogen of the α-amino group.

In general, the abbreviations used herein for designating the amino acids and the protective groups are based on recommendations of the IUPAC-IUB Commision of Biochemical Nomenclature, see European Journal of Biochemistry 138, 9 (1984). For instance, Gly, Val, Thr, Ala, Ile, Asp, Ser and Leu represent the residues of glycine, L-valine, L-threonine, L-alanine, L-isoleucine, L-aspartic acid, L-serine and L-leucine, respectively.

The asymmetric carbon atoms residing in the principal linear axis (i.e. the backbone) of the peptides of formula 1, exclusive of the terminal groups, have an S configuration. Asymmetric carbon atoms residing in the side chain of an amino acid or derived amino acid residue, including those in terminal groups, may also have the R configuration. Furthermore, with respect to disubstituted benzoyl and disubstitued phenyl(1-10C)alkanoyl as defined for X of peptides of formula 1, the substituents are selected on the basis that they do not interfere with each others presence.

The term 'halo' as used herein means a halo radical selected from bromo, chloro, fluoro or iodo.

The term "lower alkyl" as used herein, either alone or in combination with a radical, means straight chain alkyl radicals containing one to six carbon atoms and branched chain alkyl radicals containing three to six carbon atoms and includes methyl, ethyl, propyl, butyl, hexyl, 1-methylethyl, 1-methylpropyl, 2-methylpropyl and 1,1-dimethylethyl.

The term "lower cycloalkyl" as used herein, either alone or in combination with a radical, means saturated cyclic hydrocarbon radicals containing three to six carbon atoms and includes cyclopropyl, cyclobutyl, cyclopentyl and cyclohexyl.

The term "lower alkoxy" as used herein means straight chain alkoxy radicals containing one to four carbon atoms and branched chain alkoxy radicals containing three to four carbon atoms and includes methoxy, ethoxy, propoxy, 1-methylethoxy, butoxy and 1,1-dimethylethoxy. The latter radical is known commonly as tertiary-butyloxy.

The term "(1-10C)alkoxy" as used herein, either alone or in combination with a radical, means straight and branched chain alkoxy radicals containing from one to ten carbon atoms. The term "(1-10C)alkanoyl" as used herein means straight or branched chain 1-oxoalkyl radicals containing for one to ten carbon atoms; for example, acetyl, 4-methyl-1-oxopentyl (or 4-methylpentanoyl) or 1-oxooctyl (or octanoyl). The term "phenyl(2-10)alkanoyl" as used herein means

phenyl substituted 1-oxoalkyl radicals wherein the 1-oxoalkyl portion thereof is a straight or branched chain 1-oxoalkyl containing from two to ten carbon atoms; for example, 1-oxo-3-phenylpropyl and 1-oxo-5-methyl-6-phenylhexyl.

The symbol "ψ[CSNH]" used between the three-letter representations of two amino acid residues means that the normal amide bond between those residues in the peptide, being represented, has been replaced with a thioamide bond.

The term "pharmaceutically acceptable carrier" or "veterinarily acceptable carrier" as use herein means a non-toxic, generally inert vehicle for the active ingredient which does not adversely affect the ingredient.

The term "physiologically acceptable carrier" as used herein means an acceptable cosmetic vehicle of one or more non-toxic excipients which do not react with or reduce the effectiveness of the active ingredient contained therein.

The term "veterinarily acceptable carrier" as used herein means a physiologically acceptable vehicle for administering drug substances to domestic animals comprising one or more non-toxic pharmaceutically acceptable excipients which do not react with the drug substance or reduce its effectiveness.

The term "effective amount" means a predetermined antiviral amount of the antiviral agent, i.e. an amount of the agent sufficient to be effective against the viral organisms in vivo.

The term "coupling agent" as used herein means an agent capable of effecting the dehydrative coupling of an amino acid or peptide free carboxy group with a free amino group of another amino acid or peptide to form an amide bond between the reactants. The agents promote or facilitate the dehydrative coupling by activating the carboxy group. Descriptions of such coupling agents and activated groups are included in general text books of peptide chemistry; for instance, E. Schroder and K.L. Lubke, "The Peptides", Vol. 1, Academic Press, New York, N.Y., 1965, pp 2-128, and K.D. Kopple, "Peptides and Amino acids", W.A. Benjamin, Inc., New York, N.Y., 1966, pp 33-51. Examples of coupling agents are thionyl chloride, diphenylphosphoryl azide, 1,1′-carbonyldiimidazole, dicyclohexylcarbodiimide, N-hydroxysuccinimide, or 1-hydroxybenzotriazole in the presence of dicyclohexylcarbodiimide. A very practical and useful coupling agent is (benzotriazol-1-yloxy)tris(dimethylamino)-phosphonium hexafluorophosphate, described by B. Castro et al., Tetrahedron Letters, 1219 (1975), see also D. Hudson, J. Org. Chem., 53, 617 (1988), either by itself or in the presence of 1-hydroxybenzotriazole.

Process

The peptides of formula 1 can be prepared by processes which incorporate therein methods commonly used in peptide synthesis such as classical solution coupling of amino acid residues and/or peptide fragments, and if desired solid phase techniques. Such methods are described, for example, by E. Schroder and K. Lubke, cited above, in the textbook series, "The Peptides: Analysis, Synthesis, Biology", E. Gross et al., Eds., Academic Press, New York, N.Y., 1979-1987, Volumes 1 to 8, and by J.M. Stewart and J.D. Young in "Solid Phase Peptide Synthesis", 2nd ed., Pierce Chem. Co., Rockford, IL, USA, 1984.

A common feature of the aforementioned processes for the peptides is the protection of the reactive side chain groups of the various amino acid residues or derived amino acid residues with suitable protective groups which will prevent a chemical reaction from occurring at that site until the protective group is ultimately removed. Usually also common is the protection of an α-amino group on an amino acid or a fragment while that entity reacts at the carboxy group, followed by the selective removal of the α-amino protective group to allow subsequent reaction to take place at that location. Usually another common feature is the initial protection of the C-terminal carboxyl of the amino acid residue or peptide fragment, if present, which is to become the C-terminal function of the peptide, with a suitable protective group which will prevent a chemical reaction from occurring at that site until the protective group is removed after the desired sequence of the peptide has been assembled.

In general, therefore, a peptide of formula 1 can be prepared by the stepwise coupling in the order of the sequence of the peptide of the amino acid or derived amino acid residues, or fragments of the peptide, which if required are suitably protected, and eliminating all protecting groups, if present, at the completion of the stepwise coupling to obtain the peptide of formula 1. More specific processes are illustrated in the examples hereinafter.

With reference to the preparation of peptides of formula 1 in which Z is 5-1H-tetrazolyl, the derived amino acid residue containing the tetrazole can be prepared as follows: Boc-Leu-$NH_2$, for example, was converted to its corresponding nitrile derivative by treatment with p-toluenesulfonyl chloride in methylenedichloride in the presence of excess pyridine and a catalytic amount of 4-dimethylaminopyridine (Fieser and Fieser, "Reagents for Organic Synthesis, John Wiley and Sons, Inc., New York, NY, USA, 1967, vol 1, p 1183). The nitrile derivative then was mixed with tributyl tin azide, J.G.A. Luijten et al.; Rec. Trav., 81, 202 (1962), giving a tetrazole tin intermediate [cf. K. Sisido et al., Journal of Organometallic Chemistry, 33, 337 (1971) and J. Dubois et al., J. Med. Chem., 27, 1230 (1984)]. The latter was treated with hydrogen chloride in diethyl ether to afford the desired tetrazole residue as a hydrochloride salt, for example, $NH_2CH[CH_2CH(CH_3)_2]$-5-1H-tetrazole dihydrochloride. The tetrazole residue, or its hydrochloride salt, was used for coupling with the appropriate amino acid or protected fragment, leading to the desired peptide of formula 1.

With reference to the preparation of peptides of formula 1 in which Y is 5-1H-tetrazolyl, the required tetrazole-containing derived amino acid residue, preferably, N-(tertiary-butyloxycarbonyl)-2(S)-amino-3-(5-1H-tetrazolyl)-propionic acid benzyl ester, was prepared in a similar fashion starting from commercially available N-(tertiary-butyloxycarbonyl)-2(S)-amino-3-cyanopropionic acid benzyl ester. Alternatively, a derived amino acid having a cyanomethyl side chain, e.g. N-(tertiary-butyloxycarbonyl)-2(S)-amino-3-cyanopropionic acid, can be coupled with other amino acid residues to give a protected pentapeptide intermediate. Subsequent transformation of the cyano group of the intermediate to a tetrazole is effected by treating the intermediate with tributyl tin azide. Thereafter, subsequent deprotection of the product affords the corresponding peptide of formula 1 in which Y is 5-1H-tetrazolyl.

N-alkylated amino acid residues required for the preparation of peptides of formula 1 in which $R^1$ and/or $R^4$ are lower alkyl or phenyl(lower)alkyl are commercially available, such as N-(tertiary-butyloxycarbonyl)-N-methylvaline, or can be prepared by known processes. For example, N-(tertiary-butyloxycarbonyl)-N-(alkyl or phenylalkyl)-valine residues can be prepared by the method of S.T. Cheung and L. Benoiton, Can. J. Chem., 55, 916 (1977) involving a reductive amination process using sodium cyanoborohydride, L-valine benzyl ester tosylate and the corresponding aldehyde. Tertiary-butyl-oxycarbonyl-N-methyl-amino acids also can be prepared by the method of R.K. Olsen, J. Org. Chem., 35, 1912 (1970) using methyl iodide and silver oxide.

The peptide of formula 1 of this invention can be obtained in the form of a therapeutically acceptable salt.

In the instance where a particular peptide has a residue which functions as a base, examples of such salts are those with organic acids, e.g. acetic, lactic, succinic, benzoic, salicylic, methanesulfonic or p-toluenesulfonic acid, as well as polymeric acids such as tannic acid or carboxymethyl cellulose, and also salts with inorganic acids such as hydrohalic acids, e.g. hydrochloric acid, or sulfuric acid, or phorphoric acid. If desired, a particular acid addition salt is converted into another acid addition salt, such as a non-toxic, pharmaceutically acceptable salt, by treatment with the appropriate ion exchange resin in the manner described by R.A. Boissonnas et al., Helv. Chim. Acta, 43, 1849 (1960).

In the instance where a particular peptide has one or more free carboxy groups, examples of such salts are those with the sodium, potassium or calcium cations, or with strong organic bases, for example, triethylamine or N-methylmorpholine.

## Antiherpes Activity

The antiviral activity of the peptides of formula 1 can be demonstrated by biochemical, microbiological and biological procedures showing the inhibitory effect of the compounds on the replication of herpes simplex viruses, types 1 and 2 (HSV-1 and HSV-2), and other herpes viruses, for example, varicella zoster virus (VZV), Epstein-Barr virus (EBV), equine herpes virus (EHV) and cytomegalovirus.

Noteworthy is the fact that all of the aforementioned viruses are dependent on their own ribonucleotide reductase to synthesize deoxyribonucleotides for their replication. Although this fact may not be directly linked with the antiviral activity found for the present peptides the latter compounds have been shown so far to have antiviral properties against all viruses dependent on ribonucleotide reductase to synthesis DNA for their replication.

In the examples hereinafter, the inhibitory effect on herpes ribonucleotide reductase is noted for exemplary peptides of formula 1. Noteworthy, in the connection with this specific inhibition of herpes ribonucleotide reductase, is the relatively minimal effect or absence of such an effect of the peptides on cellular ribonucleotide reductase activity required for normal cell replication.

A method for demonstrating the inhibitory effect of the peptides of formula 1 on viral replication is the cell culture technique; see, for example, T. Spector et al., Proc. Natl. Acad. Sci. USA, 82, 4254 (1985).

The therapeutic effect of the peptides can be demonstrated in laboratory animals, for example, by using an assay based on genital herpes infection in Swiss Webster mice, described by E.R. Kern, et al., Antiviral Research, 3, 253 (1983).

When a peptide of this invention, or one of its therapeutically acceptable salts, is employed as an antiviral agent, it is administered topically or systemically to warm-blooded animals, e.g. humans, pigs or horses, in a vehicle comprising one or more pharmaceutically acceptable carriers, the proportion of which is determined by the solubility and chemical nature of the peptide, chosen route of administration and standard biological practice. For topical administration, the peptide can be formulated in pharmaceutically accepted vehicles containing 0.1 to 10 percent, preferably 0.5 to 5 percent, of the active agent. Such formulations can be in the form of a solution, cream or lotion.

For systemic administration, the peptide of formula 1 is administered by either intravenous, subcutaneous or intramuscular injection, in compositions with pharmaceutically acceptable vehicles or carriers. For administration by injection, it is preferred to use the peptide in solution in a sterile aqueous vehicle which may also contain other solutes such as buffers or preservatives as well as sufficient quantities of pharmaceutically acceptable salts or of glucose to make the solution isotonic.

Suitable vehicles or carriers for the above noted formulations are described in standard pharmaceutical texts, e.g. in "Remington's Pharmaceutical Sciences", 16th ed, Mack Publishing Company, Easton, Penn., 1980.

The dosage of the peptide will vary with the form of administration and the particular active agent chosen. Furthermore, it will vary with the particular host under treatment. Generally, treatment is initiated with small increments until the optimum effect under the circumstances is reached. In general, the peptide is most desirably administered at a concentration level that will generally afford antivirally effective results without causing any harmful or deleterious side effects.

With reference to topical application, the peptide is administered cutaneously in a suitable topical formulation to the infected area of the body e.g. the skin or part of the oral or genital cavity, in an amount sufficient to cover the infected area. The treatment should be repeated, for example, every four to six hours until lesions heal. Healing results usually within 3 to 4 days. No contraindications have been observed.

With reference to systemic administration, the peptide of formula 1 is administered at a dosage of 10 mcg to 1000 mcg per kilogram of body weight per day, although the aforementioned variations will occur. However, a dosage level that is in the range of from about 100 mcg to 500 mcg per kilogram of body weight per day is most desirably employed in order to achieve effective results.

Another aspect of this invention comprises a cosmetic composition comprising a herpes viral prophylactic amount of the peptide of formula 1, or a therapeutically acceptable salt thereof, together with a physiologically acceptable cosmetic carrier. Additional components, for example, skin softeners, may be included in the formulation. The cosmetic formulation of this invention is used prophylactically to prevent the outbreak of herpetic lesions of the skin. The formulation can be applied nightly to susceptible areas of the skin. Generally, the cosmetic composition contains less of the peptide than corresponding pharmaceutical compositions for topical application. A preferred range of the amount of the peptide in the cosmetic composition is 0.01 to 0.2 percent by weight.

Although the formulation disclosed hereinabove are effective and relatively safe medications for treating herpes viral infections, the possible concurrent administration of these formulations with other antiviral medications or agents to obtain beneficial results is not excluded. Such other antiviral medications or agents include acyclovir and antiviral surface active agents or antiviral interferons such as those disclosed by S.S. Asculai and F. Rapp in U.S. patent 4,507,281, March 26, 1985.

The following examples illustrate further this invention. Solution percentages or ratios express volume to volume relationship, unless stated otherwise. Abbreviations used in the examples include Boc: t-butyloxycarbonyl; BOP: (benzotriazol- 1-yloxy)tris(dimethylamino)-phosphonium hexafluorophosphate; iBu: 2-methylpropyl; Bzl: benzyl; $CH_2Cl_2$: methylenedichloride; DAT: desaminotyrosyl or 1-oxo-3-(4-hydroxyphenyl)propyl; DIPEA: diisopropylethylamine; DCC: N,N-dicyclohexylcarbodiimide; DMF: dimethyl formamide; Et: ethyl; $Et_2O$: diethyl ether; EtOH: ethanol; HOBt: 1-hydroxybenzotriazole; HPLC: high performance liquid chromatography: Me: methyl; MeOH: methanol; N-Me-Val: N-methylvalyl residue; $PhCH_2CH_2CO$: 1-oxo-3-phenylpropyl; $PhCH_2CH_2CH_2CO$: 1-oxo-4-phenylbutyl; TFA: trifluoroacetic acid. Temperatures are given in degrees centrigrade.

Example 1

General Procedure for the Solid Phase Preparation of Formula 1

A modified version of the solid phase method of R.B. Merrifield, J. Am. Chem. Soc., 85, 2149 (1963) was used to prepare the peptides using preferably a BHA-photoresin such as [4-(2-chloropropionyl)phenoxy]acetamidomethyl-copoly(styrene-1% divinyl-benzene) resin, see D. Bellof and M. Mutter, Chemia, 39,317 (1985). Protection of free carboxy groups and hydroxy groups was provided by the Bzl protective group. Typically, a Boc-amino acid, representing the C-terminal unit of the desired peptide, e.g. Boc-Leu-OH, was linked to the above-noted photoresin by the potassium fluoride method of K. Horiki et al., Chem. Lett., 165 (1978), using 9 molar equivalents of KF and 3.6 molar equivalents of Boc-Leu-OH, for example in DMF at 70° C for 24 hours, to give [4-{2-(Boc-leucine)-propionyl}phenoxy]acetamidomethyl-copoly(styrene-1% divinylbenzene) resin. The dried amino acid-solid support typically showed a leucine content of 0.6 to 0.8 mmol/g for the product, as determined by deprotection of an aliquot, followed by picric acid titration, B.F. Gisin, Anal. Chim. Acta, 58, 248 (1972). The latter amino acid-solid support was used to build up the required sequence of units (i.e. amino acid residues, derived amino acid residues) of the desired peptide by solid phase methodology. Two molar equivalents (per mole of the amino acid-solid support) of the appropriate amino acid residues were coupled serially to the solid support system using BOP (2 molar equivalents), or BOP (2 molar equivalents)/HOBt (1 molar equivalent), in the presence of N-methylmorpholine (6 molar equivalents) in dry DMF. Completion of coupling was verified by a negative ninhydrin test, E. Kaiser et al., Anal Biochem., 34, 595 (1979). Double coupling was used when necessary.

Cleavage of the protected peptide from the solid support was accomplished by irradiation at 330 nm in EtOH/DMF (1:4) at 0° under an argon atmosphere for 6 to 18 h. Protective groups (Bzl), if present, were removed from the cleavage product sby hydrogenolysis over 5% or 10% Pd/C or 20% $Pd(OH)_2/C$ by standard procedures (cf. example 1). Purification of the final product was performed by reversed-phase HPLC to better than 95% homogeneity using 0.06% aqueous TFA/acetonitrile gradients.

More specifically exemplified, the protected peptide, DAT-N-Me-Val-Ile-Asn-Asp(OBzl)-Leu-OH was assembled by the preceding procedure on a BHA photoresin using BOP/HOBt as the coupling agent, followed by cleavage of the resulting protected peptide resin by photolysis under argon at -5° for 6 h. DMF:EtOH (4:1) was used as the photolysis medium. Deprotection of the cleavage product was effected by hydrogenolysis using 5% Pd/C as catalyst. Purification of the product was done by HPLC, the product being dissolved in O.1N aqueous $NH_4OH$ and the solution adjusted to pH6 with O.1N aqueous AcOH. Whatman Partisil ® 100DS-3 C-18 column (2.2 X 50cm$^2$), 10 micron particle size, was used. Elution was done with gradients of acetonitrile and 0.06% aqueous TFA. Pure fractions (determined by analytical HPLC) were pooled and lyophilized to give DAT-N-Me-Val-Ile-Asn-Asp-Leu-OH. MS: 735 (M + H)$^+$.

The above procedure was used to prepare the peptides listed in the table of example 5. Commercially available Boc-amino acids were used. Unnatural amino acids were used in their Boc protected form; they were either commercially available, readily prepared from commercially available corresponding amino acids by reaction with di-tertiary-butyl carbonate, or prepared by standard methods. Modifications of the procedure for the preparation of terminal tetrazole peptides of formula 1 wherein Z is 5-1H-tetrazolyl and tetrazole peptides of formula 1 wherein Y is 5-1H-tetrazolyl are illustrated in the following two examples.

Example 2

Preparation of PhCH,CH,CO-N-Me-Val-Ile-Asn-Asp-NHCH[$CH_2$-CH($CH_3$)$_2$]-5-1H-tetrazole

A modified procedure of example 1 was used to assemble the protected tetrapeptide $PhCH_2CH_2$CO-N-Me-Val-Ile-Asn-Asp(OBzl)-OH wherein Boc-Asp(OBzl)-OH was linked to the photoresin and HOBt/DCC was used for coupling Boc-Asn-OH. A solution of the protected tetrapeptide (375 mg, 0.54 mmol) and 3-methyl-1(S)-(5-1H-tetrazolyl) butylamine (375 mg., 1.25 mmol) in DMF (15 ml) was cooled to 0°. DIPEA (284 mg) and then diphenylphosphoryl azide (760 mg) were added to the solution. The reaction mixture was stirred for 16 h at 0° and then partitioned between $Et_2O$ and $H_2O$ containing of few drops of concentrated $NH_4OH$ (pH=9.5). The aqueous phase was separated, washed with fresh $Et_2O$ and filtered through a 45 µm membrane. The pH of the filtrate was adjusted to pH6.5 by the addition of AcOH and again filtered through a 45 µm membrane. The filtrate was loaded on a HPLC preparatory column. The column was eluted with a continuous gradient of 0.06% aqueous TFA in acetonitrile. The pure fractions as indicated by analytical HPLC were combined and lyophilized to give the corresponding Asp(OBzl) protected peptide (132 mg) of the title compound.

The latter compound (112 mg) was subjected to hydrogenolysis at. one atmosphere of pressure at room temperature [5% Pd/C (100 mg) in EtOH (30 ml) for 16 h]. The reaction mixture was filtered and the filtrate concentrated to dryness under reduced pressure. The residue was dissolved in acetonitrile and $H_2O$. The acetonitrile was removed from the solution by distillation under reduced pressure. Lyophization of the resultant concentrate gave the title compound (88 mg) as a white powder which was 90% pure as indicated by analytical HPLC, FAB/MS: 743(M+1)$^+$.

Example 3

Preparation of $PhCH_2CH_2$CO-N-Me-Val-Ile-NHCH[$CH_2$-5-1H-tetrazole]CO-Asp-Leu-OH

The intermediate $PhCH_2CH_2$CO-N-Me-Val-Ile-NHCH-($CH_2$CN)CO-Asp-Leu-OH was prepared by following the procedure of example 1 and the appropriate Boc amino acids (including the commercially available Boc-NHCH($CH_2$CN) COOH). All couplings were done with BOP/HOBt. The intermediate (40 mg) and tributyltin aside (100 mg) in anhydrous tetrahydrofuran were heated in a sealed tube at 80 to 90 for 4 days. The tube was cooled and opened. Solvent was removed from the contents by evaporation under reduced pressure.

The residue was suspended in anhydrous $Et_2O$ and gaseous HCl was bubbled into the mixture for 45 min. After evaporation of the $Et_2O$, the solid residue was triturated with hexane, and then dissolved in EtOH. The solution was filtered and the filtrate evaporated to give 35 mg of a white solid. The solid was dissolved in $H_2O$-acetonitrile (1:1, 3.7 ml) and the solution injected into a semi-prep HPLC. Elution with gradients of 0.06% aqueous TFA and acetonitrile afforded the Asp(OBzl) protected peptide of the title compound. Deprotection of the protected peptide by hydrogenolysis according to the condition described in example 2 afforded the title compound (10.6 mg) which was 100% pure as indicated by analytical HPLC. FAB/MS: 744 (M+1)$^+$.

Example 4

Preparation of the Intermediate Boc-Asp(OBzl)Ψ[CSNH]Leu-OBzl

(An example of the procedure used to prepare thioamide intermediates for the preparation of peptides of formula

1 wherein W is thioxo.)

A stirred mixture of Boc-Asp(OBzl)Leu-OBzl (2.90g, 5.51 mmol) and Lawesson's reagent (1.12g, 2.7 mmol), see U. Pederson et al., Tetrahedron, 38, 3267 (1982), in toluene (30ml) was heated at reflux for 2h. Column chromatography with $SiO_2$ (3.5 X 30 cm) and elution with $CH_2Cl_2$ gave the title compound (2.0g), MS: 543 $(M+H)^+$, as yellow oil (major fraction).

Analogous thioamides were prepared in the same manner and incorporated into the appropriate peptides of formula 1 according to conventional solution phase peptide synthesis.

Example 5

Inhibition of Herpes Simplex Virus (HSV, type 1) Ribonucleotide Reductase

a) Preparation of Enzyme

HSV-1 ribonucleotide reductase (partially purified) was obtained from quiescent BHK-21/C13 cells infected with strain F HSV-1 virus at 10 plaque forming units/cell as described by E.A. Cohen et al., J. Gen. Virol., 66, 733 (1985).

b) Assay and Results for Exemplified Peptides

By following the procedure described by P. Gaudreau et al., J. Biol, Chem., 262, 12413 (1987), the assay results listed in the following table were obtained The assay result for each peptide is expressed as the concentration of the peptide producing 50% of the maximal inhibition ($IC_{50}$) of enzyme activity. The number of units of the enzyme preparation used in each assay was constant, based on the specific activity of the enzyme preparation. The results are relative to the activity obtained in control experiments without peptide and represent the mean of four assays that varied less than 10% with each other.

TABLE

$XNR^1$-$CH(R^2)$-CO-Ile-NH-$CH(CH_2Y)$-CO-Asp-$NHCH(R^7)$-Z (Peptides of formula 1 wherein X, $R^1$, Y and Z are shown below, $R^2$ is $CH(CH_3)_2$, $R^3$ is $CH(CH_3)C_2H_5$, $R^4$, $R^5$ and $R^6$ are hydrogen, $R^7$ is $CH_2CH(CH_3)_2$ and W is O).

| X | $R^1$ | Y | Z | FAB/MS $(M+H)^+$ | $IC_{50}$ (μM) |
|---|---|---|---|---|---|
| $CH_3CO$ | $PhCH_2CH_2$ | $CONH_2$ | COOH | 719 | 17 |
| $(CH_3)_2CHCH_2CH_2CO$ | $CH_3$ | $CONH_2$ | COOH | 685 | 7.6 |
| 2-(biphenylyl)carbonyl | $CH_3$ | $CONH_2$ | COOH | 767 | 2.5 |
| 2-(phenylmethyl) benzoyl | $CH_3$ | $CONH_2$ | COOH | 781 | 5.5 |
| $PhCH_2CH_2CH_2CO$ | $CH_3$ | $CONH_2$ | COOH | 733 | 6.2 |
| $PhCH_2CH_2CO$ | $CH_3$ | $CONH_2$ | 5-1H-t* | 743 | 3.8 |
| $PhCH_2CH_2CO$ | $CH_3$ | 5-1H-t* | COOH | 744 | 2.0 |
| DAT | $CH_3$ | $CONH_2$ | COOH | 735 | 6.0 |

*5-1H-t represents 5-1H-tetrazolyl

Other examples of the peptides of formula 1 are:

$PhCH_2CH_2CO$-N-Me-Val-Ile-Asp-Asp-Leu-OH

$PhCH_2CH_2CO$-N-Et-Val-Ile-Asn-Asp-Leu-OH

$PhCH_2CH_2CO$-N-iBu-Val-Ile-Asn-Asp-Leu-OH

$PhCH_2CH_2CO$-N-Me-Val-Ile-Asn-Asp-Leu-OH

(3,4-dihydroxy-Ph)$CH_2CH_2CO$-N-Me-Val-Ile-Asn-Asp-Leu-OH

[2-(2′-carboxy)biphenylyl]carbonyl-N-Me-Val-Ile-Asn-Asp-Leu-OH

$PhCH_2CH_2CO$-N-Me-Thr-Ile-Asn-Asp$\psi$[CSNH]Leu-OH

## Claims

**1.** A peptide of formula 1

$$XNR^1\text{-}CH(R^2)\text{-}C(O)\text{-}NH\text{-}CH(R^3)\text{-}C(O)\text{-}NR^4\text{-}CH(CH_2Y)\text{-}CO\text{-}NH\text{-}CH\text{-}[CR^5(R^6)\text{-}COOH]\text{-}C(W)\text{-}NH\text{-}CH(R^7)\text{-}Z \quad 1$$

wherein X is (1-10C)alkanoyl, (1-10C)alkoxycarbonyl, benzoyl, benzoyl monosubstituted or disubstituted with a substituent selected from halo, hydroxy, lower alkyl, lower alkoxy, phenyl, 2-carboxyphenyl or benzyl, 2,2-diphenylacetyl, phenyl(2-10C)alkanoyl or phenyl(2- 10C)alkanoyl monosubstituted or disubstituted on the aromatic portion thereof with a substituent selected from halo, hydroxy, lower alkyl, lower alkoxy or phenyl;

$R^1$ is lower alkyl or phenyl(lower)alkyl;
$R^2$ is $CH(CH_3)_2$ or $CH(OH)CH_3$;
$R^3$ is $CH(CH_3)C_2H_5$;
$R^4$, $R^5$ and $R^6$ each is hydrogen;
$R^7$ is $CH_2CH(CH_3)_2$;
W is oxo or thioxo;
Y is carboxy, carbamyl or 5-1H-tetrazolyl; and
Z is carboxy or 5-1H-tetrazolyl; or a therapeutically acceptable salt thereof;

wherein the terms "lower alkyl" and "lower alkoxy" as used herein, either alone or in combination with a radical, are defined as follows:
"lower alkyl": straight chain alkyl radicals containing one to six carbon atoms and branched chain alkyl radicals containing three to six carbon atoms;
"lower alkoxy": straight chain alkoxy radicals containing one to four carbon atoms and branched chain alkoxy radicals containing three to four carbon atoms.

**2.** A compound according to claim 1, wherein X and $R^1$ are defined in claim 1,

$R^2$ is $CH(CH_3)_2$;
$R^3$ is $CH(CH_3)C_2H_5$;
$R^4$, $R^5$ and $R^6$ each is hydrogen;
$R^7$ is $CH_2CH(CH_3)_2$;
W is oxo;

Y is carbamyl or 5-1H-tetrazolyl; and
Z is carboxy or 5-1H-tetrazolyl; or a therapeutically acceptable salt thereof.

**3.** A peptide in formula 1 of claim 1 selected from the group consisting of:

DAT-N-Me-Val-Ile-Asn-Asp-Leu-OH
$CH_3CO$-(N-phenylethyl-Val)-Ile-Asn-Asp-Leu-OH
$(CH_3)_2CHCH_2CH_2CO$-N-Me-Val-Ile-Asn-Asp-Leu-OH
2-(biphenylyl)carbonyl-N-Me-Val-Ile-Asn-Asp-Leu-OH
2-(phenylmethyl)benzoyl-N-Me-Val-Ile-Asn-Asp-Leu-OH
$PhCH_2CH_2CH_2CO$-N-Me-Val-Ile-Asn-Asp-Leu-Oh
$PhCH_2CH_2CO$-N-Me-Val-Ile-Asn-Asp-NHCH[$CH_2CH(CH_3)_2$]-5-1H-tetrazole and
$PhCH_2CH_2CO$-N-Me-Val-Ile-NHCH[$CH_2$-(5-1H-tetrazolyl)]-CO-Asp-Leu-OH.

**4.** A pharmaceutical composition comprising a peptide as recited in anyone of claims 1 to 3, or therapeutically acceptable salt thereof, and a pharmaceutically or veterinarily acceptable carrier.

**5.** A cosmetic composition comprising a peptide as recited anyone of claims 1 to 3, or a therapeutically acceptable salt thereof, and a physiologically acceptable carrier suitable for topical application.

**6.** A peptide as recited in anyone of claims 1 to 3, are therapeutically acceptable salt thereof, for use in a method of inhibiting the replication of herpes virus.

**7.** Use of a peptide as recited in anyone of claims 1 to 3, or a therapeutically acceptable salt thereof, for the preparation of a medicament for treating a herpes viral infection in a mammal.

**8.** Use according to claim 7 wherein the herpes viral infection is a herpes simplex viral infection.

**9.** Use of a peptide as recited in anyone of claims 1 to 3, or a therapeutically acceptable salt thereof, for the preparation of a medicament for inhibiting the replication of herpes virus.

**10.** A process for preparing a peptide as recited in claim 1, or a therapeutically acceptable salt thereof, comprising:

a) stepwise coupling, in the order of the sequence of the peptide, of the amino acid or derived amino acid residues, or fragments or the peptide, in which

i) reactive side chain groups of the residue or fragments are protected with suitable protective groups to prevent chemical reactions from occurring at that site until the protective group is ultimately removed after the completion of the stepwise coupling;

ii) an α-amino group of a coupling reactant is protected by an α-amino protective group while the free carboxy group of that reactant couples with the free α-amino group of the second reactant; the α-amino protective group being one which can be selectively removed to allow the subsequent coupling step to take place at that α-amino group; and

iii) the C-terminal carboxyl of the amino acid residue of the amino acid residue or peptide fragment, which is to become the C-terminal function of the protected peptide, if present, is protected with a suitable protective group which will prevent chemical reaction occurring at that site until after the desired amino acid sequence for the peptide has been assembled; and

b) at the completion of the coupling, eliminating any protecting groups to obtain the peptide of claim 1; and if desired, converting the peptide into a therapeutically acceptable salt.

## Patentansprüche

**1.** Peptid der Formel 1

$$XNR^1\text{-}CH(R^2)\text{-}C(O)\text{-}NH\text{-}CH(R^3)\text{-}C(O)\text{-}NR^4\text{-}CH(CH_2Y)\text{-}CO\text{-}NH\text{-}CH\text{-}[CR^5(R^6)\text{-}COOH]\text{-}C(W)\text{-}NH\text{-}CH(R^7)\text{-}Z \quad (1)$$

in welcher

X die Bedeutung (1-10C)Alkanoyl, (1-10C)Alkoxycarbonyl, Benzoyl, mit einem unter Halo, Hydroxy, Niederalkyl, Niederalkoxy, Phenyl, 2-Carboxyphenyl oder -benzyl, 2,2-Diphenylacetyl, Phenyl(2-10C)alkanoyl oder mit einem unter Halo, Hydroxy, Niederalkyl, Niederalkoxy oder Phenyl gewählten Substituenten auf dessen aromatischem Teil mono- oder disubstituiertes Phenyl(2-10C)alkanoyl gewählten mono- oder disubstituiertes Benzoyl hat;
$R^1$ Niederalkyl oder Phenylniederalkyl bedeutet;
$R^2$ $CH(CH_3)_2$ oder $CH(OH)CH_3$ bedeutet;
$R^3$ $CH(CH_3)C_2H_5$ bedeutet;
$R^4$, $R^5$ und $R^6$ jeweils Wasserstoff bedeuten;
$R^7$ $CH_2CH(CH_3)_2$ bedeutet;
W Oxo oder Thioxo bedeutet;
Y Carboxy, Carbamyl oder 5-1H-Tetrazolyl bedeutet; und
Z Carboxy oder 5-1H-Tetrazolyl bedeutet;

oder ein therapeutisch annehmbares Salz davon;

wobei die hier entweder allein oder in Kombination mit einem Rest verwendeten Bezeichnungen "Niederalkyl" und "Niederalkoxy" wie folgt definiert sind:
"Niederalkyl": geradkettige Alkylreste mit einem bis sechs Kohlenstoffatomen und verzweigtkettige Alkylreste mit drei bis sechs Kohlenstoffatomen;
"Niederalkoxy": geradkettige Alkoxyreste mit einem bis vier Kohlenstoffatomen und verzweigtkettige Alkoxyreste mit drei bis vier Kohlenstoffatomen.

**2.** Verbindung nach Anspruch 1, in welcher X und $R^1$ wie im Anspruch 1 definiert sind;

$R^2$ $CH(CH_3)_2$ bedeutet;
$R^3$ $CH(CH_3)C_2H_5$ bedeutet;
$R^4$, $R^5$ und $R^6$ jeweils Wasserstoff bedeuten;
$R^7$ $CH_2CH(CH_3)_2$ bedeutet;
W Oxo bedeutet;
Y Carbamyl oder 5-1H-Tetrazolyl bedeutet; und
Z Carboxy oder 5-1H-Tetrazolyl bedeutet;

oder ein therapeutisch annehmbares Salz davon.

**3.** Peptid der Formel 1, das aus der von

DAT-N-Me-Val-Ile-Asn-Asp-Leu-OH,

$CH_3CO$-(N-phenylethyl-Val)-Ile-Asn-Asp-Leu-OH,

$(CH_3)_2CHCH_2CH_2CO$-N-Me-Val-Ile-Asn-Asp-Leu-OH,

2-(Biphenylyl)carbonyl-N-Me-Val-Ile-Asn-Asp-Leu-OH,

2-(Phenylmethyl)benzoyl-N-Me-Val-Ile-Asn-Asp-Leu-OH,

$PhCH_2CH_2CH_2CO$-N-Me-Val-Ile-Asn-Asp-Leu-OH,

$PhCH_2CH_2CO$-N-Me-Val-Ile-Asn-Asp-NHCH[$CH_2CH(CH_3)_2$]-5-1H-tetrazol

und

$PhCH_2CH_2CO$-N-Me-Val-Ile-NHCH[$CH_2$-(5-1H-tetrazolyl)]-CO-Asp-Leu-OH

gebildeten Gruppe ausgewählt ist.

**4.** Arzneimittelzusammensetzung, die ein Peptid nach einem der Ansprüche 1 bis 3 oder ein therapeutisch annehmbares Salz davon sowie einen pharmazeutisch oder tierärztlich annehmbaren Träger enthält.

**5.** Kosmetikzusammensetzung, die ein Peptid nach einem der Ansprüche 1 bis 3 oder ein therapeutisch annehmbares Salz davon sowie einen physiologisch annehmbaren, zur topischen Applikation geeigneten Träger enthält.

**6.** Zur Verwendung in einem Verfahren zur Hemmung der Replikation von Herpes Virus bestimmtes Peptid nach einem der Ansprüche 1 bis 3, oder therapeutisch annehmbares Salz davon.

**7.** Verwendung eines Peptids nach einem der Ansprüche 1 bis 3 oder eines therapeutisch annehmbaren Salzes davon zur Herstellung eines Arzneimittels zur Behandlung eines Infekts durch ein Herpes Virus bei einem Säuger.

**8.** Verwendung nach Anspruch 7, bei welcher der Infekt durch ein Herpes Virus ein Infekt durch das Herpes simplex Virus ist.

**9.** Verwendung eines Peptids nach einem der Ansprüche 1 bis 3 oder eines therapeutisch annehmbaren Salzes davon zur Herstellung eines Arzneimittels zur Hemmung der Replikation von Herpes Virus.

**10.** Verfahren zur Herstellung eines Peptids nach Anspruch 1 oder eines therapeutisch annehmbaren Salzes davon, umfassend:

a) schrittweise Kupplung der Aminosäure- oder Aminosäurenderivate-Reste oder der Peptid-Fragmente in der Reihenfolge der Peptid-Sequenz, wobei:

i) reaktive Seitenkettengruppen der Reste oder Fragmente mit geeigneten Schutzgruppen geschützt werden, um zu verhindern, dass an dieser Stelle chemische Reaktionen stattfinden, bevor und bis die Schutzgruppe nach der Vollendung der schrittweisen Kupplung schliesslich entfernt wird;

ii) eine α-Aminogruppe eines Kupplungsreagens mit einer α-Amino-Schutzgruppe geschützt wird, während die freie Carboxygruppe dieses Reagens mit der freien α-Aminogruppe des zweiten Reagens eine Kupplung eingeht; wobei die α-Amino-Schutzgruppe eine solche ist, die selektiv entfernt werden kann, um zu erlauben, dass die darauffolgende Kupplung an dieser α-Aminogruppe stattfindet; und

(iii) falls vorhanden, das C-endständige Carboxyl des Aminosäure-Rests der Aminosäure-Reste oder Peptid-Fragmente, welches dazu bestimmt ist, die C-endständige Funktion des geschützten Peptids zu werden, mit einer geeigneten Schutzgruppe geschützt wird, welche dazu bestimmt ist, zu verhindern, dass an dieser Stelle eine chemische Reaktion stattfindet, bevor und bis die gewünschte Aminosäure-Sequenz

für das Peptid zusammengesetzt worden ist; und

b) bei Vollendung der Kupplung alle Schutzgruppen entfernt werden, um ein Peptid nach Anspruch 1 zu ergeben; und gegebenenfalls das Peptid in ein therapeutisch annehmbares Salz umgewandelt wird.

## Revendications

**1.** Peptide de formule 1

$$XNR^1\text{-}CH(R^2)\text{-}C(O)\text{-}NH\text{-}CH(R^3)\text{-}C(O)\text{-}NR^4\text{-}CH(CH_2Y)\text{-}CO\text{-}NH\text{-}CH\text{-}$$

$$[CR^5(R^6)\text{-}COOH]\text{-}C(W)\text{-}NH\text{-}CH(R^7)\text{-}Z \qquad 1$$

dans laquelle X est alcanoyle en $C_1$-$C_{10}$, (alcoxy en $C_1$-$C_{10}$)carbonyle, benzoyle, benzoyle monosubstitué ou disubstitué avec un substituant choisi parmi halogéno, hydroxyle, alkyle inférieur, alcoxy inférieur, phényle, 2-carboxyphényle ou benzyle, 2,2-diphénylacétyle, phényl(alcanoyle en $C_2$-$C_{10}$) ou phényl(alcanoyle en $C_2$-$C_{10}$) monosubstitué ou disubstitué sur sa partie aromatique avec un substituant choisi parmi halogéno, hydroxyle, alkyle inférieur, alcoxy inférieur ou phényle;

$R^1$ est alkyle inférieur ou phényl(alkyle inférieur);
$R^2$ est $CH(CH_3)_2$ ou $CH(OH)CH_3$;
$R^3$ est $CH(CH_3)C_2H_5$;
$R^4$, $R^5$ et $R^6$ sont chacun l'hydrogène;
$R^7$ est $CH_2CH(CH_3)_2$;
W est oxo ou thioxo;
Y est carboxyle, carbamyle ou 5-1H-tétrazolyle, et
Z est carboxyle ou 5-1H-tétrazolyle; ou sel thérapeutiquement acceptable de celui-ci; où les termes "alkyle inférieur" et "alcoxy inférieur" tels qu'ils sont utilisés ici, seuls ou en combinaison avec un radical, sont définis de la manière suivante:

"alkyle inférieur": radicaux alkyle linéaires contenant un à six atomes de carbone et radicaux alkyle ramifiés contenant trois à six atomes de carbone;
"alcoxy inférieur": radicaux alcoxy linéaires contenant un à quatre atomes de carbone et radicaux alcoxy ramifiés contenant trois à quatre atomes de carbone.

**2.** Composé selon la revendication 1, dans lequel X et $R^1$ sont définis dans la revendication 1,

$R^2$ est $CH(CH_3)_2$;
$R^3$ est $CH(CH_3)C_2H_5$;
$R^4$, $R^5$ et $R^6$ sont chacun l'hydrogène;
$R^7$ est $CH_2CH(CH_3)_2$;
W est oxo;
Y est carbamyle ou 5-1H-tétrazolyle, et
Z est carboxyle ou 5-1H-tétrazolyle; ou sel thérapeutiquement acceptable de celui-ci.

**3.** Peptide de formule 1 selon la revendication 1, choisi dans le groupe consistant en:

DAT-N-Me-Val-Ile-Asn-Asp-Leu-OH

$CH_3CO$-(N-phényléthyl-Val)-Ile-Asn-Asp-Leu-OH

$(CH_3)_2CHCH_2CH_2CO$-N-Me-Val-Ile-Asn-Asp-Leu-OH

2-(biphénylyl)carbonyl-N-Me-Val-Ile-Asn-Asp-Leu-OH

2-(phénylméthyl)benzoyl-N-Me-Val-Ile-Asn-Asp-Leu-OH

$PhCH_2CH_2CH_2CO$-N-Me-Val-Ile-Asn-Asp-Leu-OH

$PhCH_2CH_2CO$-N-Me-Val-Ile-Asn-Asp-NHCH[$CH_2CH(CH_3)_2$]-5-1H-tétrazole et

$PhCH_2CH_2CO$-N-Me-Val-Ile-NHCH[$CH_2$-(5-1H-tétrazolyl)]-CO-Asp-Leu-OH.

**4.** Composition pharmaceutique comprenant un peptide selon l'une quelconque des revendications 1 à 3, ou un sel therapeutiquement acceptable de celui-ci, et un véhicule acceptable du point de vue pharmaceutique ou vétérinaire.

**5.** Composition cosmétique comprenant un peptide selon l'une quelconque des revendications 1 à 3, ou un sel thérapeutiquement acceptable de celui-ci, et un véhicule acceptable du point de vue physiologique approprié pour une application topique.

**6.** Peptide selon l'une quelconque des revendications 1 à 3, ou sel thérapeutiquement acceptable de celui-ci, destiné à être utilisé dans un procédé pour inhiber la réplication d'un virus de l'herpès.

**7.** Utilisation d'un peptide selon l'une quelconque des revendications 1 à 3, ou d'un sel thérapeutiquement acceptable de celui-ci, pour la préparation d'un médicament pour traiter une infection par un virus de l'herpès chez un mammifère.

**8.** Utilisation selon la revendication 7 dans laquelle l'infection par un virus de l'herpès est une infection par un virus de l'herpès simplex.

**9.** Utilisation d'un peptide selon l'une quelconque des revendications 1 à 3, ou d'un sel thérapeutiquement acceptable de celui-ci, pour la préparation d'un médicament pour inhiber la réplication de virus de l'herpès.

**10.** Procédé de préparation d'un peptide selon la revendication 1, ou d'un sel thérapeutiquement acceptable de celui-ci, comprenant:

a) le couplage par étapes, dans l'ordre de la séquence du peptide, des résidus d'acides aminés ou d'acides aminés dérivés, ou fragments du peptide, dans lequel

i) les groupes réactifs des chaînes latérales des résidus ou fragments sont protégés avec des groupes protecteurs appropriés pour empêcher des réactions chimiques de se produire à ce site jusqu'à ce que le groupe protecteur soit finalement retiré après l'achèvement du couplage par étapes;

ii) un groupe $\alpha$-amino d'un corps réagissant de couplage est protégé par un groupe $\alpha$-amino-protecteur tandis que le groupe carboxyle libre de ce corps réagissant se couple avec le groupe $\alpha$-amino libre du second corps réagissant; le groupe $\alpha$-amino-protecteur étant un groupe qui peut être retiré sélectivement pour permettre à l'étape de couplage subséquente de se dérouler au niveau de ce groupe $\alpha$-amino; et

iii) le carboxyle C-terminal du résidu d'acide aminé ou du fragment de peptide, qui doit devenir la fonction C-terminale du peptide protégé, s'il est présent, est protégé avec un groupe protecteur approprié qui empêche une réaction chimique de se produire à ce site jusqu'à ce que la séquence d'acides aminés souhaitée pour le peptide ait été assemblée; et

b) à l'achèvement du couplage, l'élimination de tout groupe protecteur pour obtenir le peptide selon la revendication 1, et, si on le souhaite, la conversion du peptide en un sel thérapeutiquement acceptable.